# EUROPEAN PATENT SPECIFICATION

(11) **EP 2 206 505 B1**
(45) Date of publication and mention of the grant of the patent: **31.07.2013**
(21) Application number: 08790627.7
(22) Date of filing: 26.06.2008
(51) Int. Cl.: A61K 35/74, A61K 9/19

(54) **NANO-LEVEL LACTIC ACID BACTERIUM**
NANOMASSSTAB-MILCHSÄUREBAKTERIUM
BACTÉRIE D'ACIDE LACTIQUE À L'ÉCHELLE NANO

(43) Date of publication of application: 14.07.2010
(73) Proprietor: Shinwa Pharmaceutical Co., Ltd., Toyama 939-2723 (JP); Bio-Lab Ltd., Saitama 350-1325 (JP); The Japanese Association Of Clinical Research on Supplements, Saitama 350-1243 (JP); Cosmo Foods Co., Ltd., Chuo-ku Tokyo 103-0001 (JP)
(72) Inventor: HASEGAWA, Hideo, Hidaka-shi Saitama 350-1243 (JP); KAN, Tatsuhiko, Sayama-shi Saitama 350-1325 (JP)
(74) Representative: Stoner, Gerard Patrick
(86) International application number: PCT/JP2008/061612
(87) International publication number: WO 2009/157073

(56) References cited:
- EP-A1- 2 149 309
- JP-A- 7 313 140
- JP-A- 8 259 450
- JP-A- 9 188 627
- JP-A- 50 132 155
- JP-A- 63 022 179
- JP-A- 2003 171 292
- JP-A- 2007 195 415
- JP-A- 2008 195 631
- US-A- 5 338 682
- US-A- 5 556 785
- US-A- 5 662 900
- KOKKINOS A. ET AL: "Cell size of various lactic acid bacteria as determined by scanning electron microscope and image analysis1-", LAIT, vol. 78, 1998, pages 491-500, XP002636246,
- 'The Science and Technology of Lactic Acid Bacteria',

## Description

### TECHNICAL FIELD

This invention relates to nano-sized lactic acid bacteria having activity to enhance interferon-α producing capacity from antigen presenting cells.

### BACKGROUND ART

According to recent research, naive T cells (hereinafter abbreviated as "Th0") are known to differentiate into type-I T cells (hereinafter abbreviated as "Th1") and type-II T cells (hereinafter abbreviated as "Th2") depending upon their functions.
An immune response by Th1 cells induces cellular immunity, leading to the occurrence of phagocytosis by mononuclear cells such as macrophages and lymphocytes. An immune response by Th2 cells, on the other hand, induces humoral immunity, so that bacterial killing by an antibody takes place. Th1 cytokine suppresses Th2, and conversely, Th2 cytokine suppresses Th1. These two cytokines act synergistically to maintain a balance in overall immune.

Interferon-α (IFN-α) is a cytokine secreted from dendritic cells (antigen presenting cells) upon infection by virus, intercellular facultative bacteria such as tubercle bacillus, salmonella, listeria or Mycobacterium laprae, or intercellular facultative fungus such as Cryptococcus.
On the other hand, interleukin 12 (IL-12) is a cytokine secreted from antigen presenting cells such as dendritic cells or macrophages, and is known as a very strong immunoactive agent that activates natural killer cells (NK cells), LAK cells (LAK cells) or killer T cells (CTL cells), which directly attack cancer cells, and enhances the production of IFN-γ.

These IFN-α and IL-12 are both cytokines that induce Th1 cells, but differences are found in receptors (TRL: Toll-like receptors) to be expressed on antigen presenting cells (TLR1, TLR3, TLR5 and TLR9 in the case of IL-12; TLR7 and TLR9 in the case of IFN-α).

Reported as lactic acid bacteria, which enhance the production of IFN-α from antigen presenting cells, and their constituents include a Lactobacillus brevis cell powder (see Patent Document 1: JP-A 6-206826), lactic acid bacteria belonging to the genus of Enterococcus and its processed product (see Patent Document 2: JP-A 8-259450), a constituent extract of Lactobacillus brevis FERM BP-4693 (see Patent Document 3: JP-A 9-188627), and so on.

If it were normal, it would be natural to choose a strain having high IFN-α producing capacity, and it would be unnecessary to dare to modify a low-activity strain into a high-activity strain, to say nothing of making such an attempt.
For business entities that have supplied Lactobacillus brevis cells to the market over many years, however, it is an urgent desire to enhance the IFN-α producing capacity of lactic acid bacteria.

Lactic acid bacteria have heretofore been normally ingested in the form of fermented milk or yoghurt. An orally-ingested antigen is taken up as a result of its phagocytosis by M cells overlying Peyer's patches.
Interested in up to what size an antigen would be taken up through Peyer's patches, a great deal of research has been conducted to date as to a correlation between the size (particle size) of particles and their uptake into Peyer's patches.

As a result, it has already been found that the phagocytosis by M cells becomes significantly low when the size of particles exceeds 10 µm (see Non-patent Document 1) and also that the maximum size of particles which pass through Peyer's patches is 10 µm when the material of the particles is polylactide (see Non-patent Document 2), 15 µm when the material of the particles is polystyrene (see Non-patent Document 3) and 21 µm when the material of the particles is biodegradable polylactic acid (see Non-patent Document 4).
It is appreciated from these results that the size of particles which can pass through Peyer's patches is as high as 20 µm.

According to an experiment in which antigen-sensitized beads of biodegradable polylactic acid were orally administered to rats, the particle size at which the percentage of production of an antigen-specific antibody as a result of induction by Th2 was 4 µm in the case of IgG and 7 µm in the case of IgA. It is, therefore, appreciated that the particle size preferable for induction by Th2 is from 3 to 7 µm or so (see Non-patent Document 4).
Nonetheless, the particle size preferable for induction by Th1 has not been reported yet to date.

Patent Document 1: JP-A 6-206826
Patent Document 2: JP-A 8-259450
Patent Document 3: JP-A 9-188627
Non-patent Document 1: Tabata Y, Ikeda Y., Adv. Polym. Sci., 94, 107-141 (1990)
Non-patent Document 2: Eldridge J.H., et al., J. Controlled Rel., 11, 205-214 (1990)
Non-patent Document 3: Eldridge J.H., et al., Molec. Immun., 28, 287-294 (1991)
Non-patent Document 4: Tabata Y., et al., Vaccine 14, 1677-1685 (1996)

### DISCLOSURE OF THE INVENTION

### PROBLEMS TO BE SOLVED BY THE INVENTION

With the foregoing circumstances in view, the present invention has as an object thereof the provision of lactic acid bacteria having a particle size preferable for induction by Th1, excellent INF-α producing capacity and superb dispersibility in water.

### MEANS FOR SOLVING THE PROBLEMS

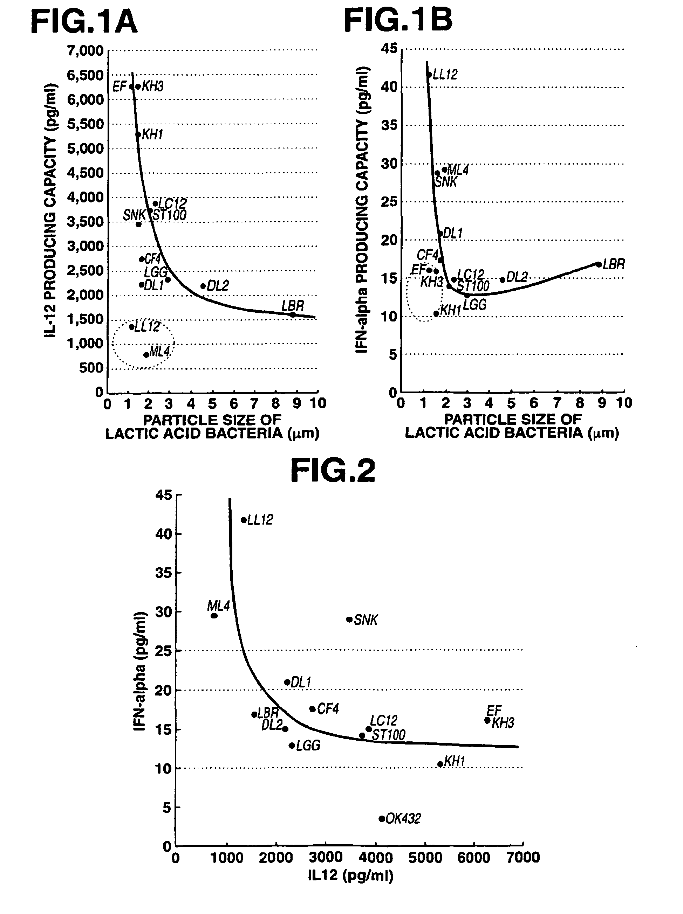

The present inventors isolated lactic acid bacteria, which contribute to longevity, from "Sugukizuke" (pickled leaves of suguki, kind of turnip) in Kyoto, "Sunkizuke" (pickled leaves of sunki, kind of red beat) in Nagano, Mariami and Matsoni-like in Republic of Georgia, fermented mare's milk in Mongolia, and various fermented dairy products, and analyzed correlations between the sizes of those lactic acid bacteria and their capacities to produce IL-12 and IFN-α from antigen presenting cells.
As a result, the IL-12 producing capacity from the antigen presenting cells as a result of stimulation by lactic acid bacteria, as shown in FIG. 1A, increased when the size of lactic acid bacteria became smaller to 1 µm or further (see Referential Example 1).
In the case of IFN-α producing capacity, on the other hand, the IFN-α producing capacity, as illustrated in FIG. 1B, also increased when the size of lactic acid bacteria became smaller to 1 µm or further (see Referential Example 1).

Some strains were, however, found to show a negative correlation between IL-12 producing capacity and IFN-α producing capacity. For example, strains such as EF, KH1 and KH3 are high in IL-12 producing capacity but low in IFN-α producing capacity. In contrast, strains such as LL12 and ML4 are low in IL-12 producing capacity but high in IFN-α producing capacity.
On the other hand, SNK has been found to have such a characteristic feature that both of its IL-12 producing capacity and IFN-α producing capacity are high relatively.
The foregoing findings are not conceivable from the related art (Japanese Patent Application No. 2007-30324), and may presumably be attributed to differences in the recognition of the lactic acid bacteria by receptors expressed on antigen presenting cells.

As a result, it was found that lactic acid bacteria, the particle size of which is as large as close to 9 µm like Lactobacillus brevis FERM BP-4693 (indicated as "LBR" in Table 1 and FIGS. 1A, 1B and 2), is far inferior in IFN-α producing capacity to one having a particle size of 1 µm or so.

To solve the above-described problem, the present inventors have then enthusiastically conducted an investigation for conditions that provide lactic acid cells with a smaller mode value in their particle size distribution and prevents reaggregation of cells themselves.
In the course of the investigation, the present inventors paid attention to the fact that lactic acid bacteria is charged positive (plus) at surfaces thereof, and found that the mode value (which may hereinafter be simply called "the particle size") in the particle size distribution of lactic acid bacteria cells can be reduced to 1.0 µm or smaller by controlling pH to a neutral range in a culturing step and processing steps.
In general, it is known that the form of lactic acid bacteria changes depending on various conditions during its culture. However, it was not conventionally known among those skilled in the present art the finding that the size of lactic acid bacteria can be adjusted to 1.0 µm or smaller by controlling pH in a culturing step and processing steps.

The present inventors also found that the capacity to produce IFN-α from antigen presenting cells can be significantly enhanced by such cells.
As has been described above, the present inventors found that even for usual lactic acid bacterial cells having a particle size greater than 1.0 µm, their particle size can be adjusted to 1.0 µm or smaller through its culture and processing at pHs in a neutral range and also that this lactic acid bacteria can enhance the IFN-α producing capacity from antigen presenting cells and can improve the immunopotentiating activity. These findings have then led to the completion of the present invention.

Recently, concentrated dry powders of dead cells of lactic acid bacteria have become increasingly available on the market. They are intended to enable the ingestion of a large number of lactic acid bacteria with a small amount.
Even if the particle size of the lactic acid bacteria itself in such a dry powder is 1.0 µm or smaller, its particles, when poured into water, undergo mutual adsorption and aggregation into clumps if it is a powder obtained by simply drying concentrated cells (see Referential Example 2).
As a matter of fact, the possibility of existence of lactic acid bacteria the particle size of which is 1.0 µm or smaller is low under usual culture conditions (see Table 1).

Described specifically, the present invention provides:
1. Cells of nano-sized lactobacillus lactic acid bacteria, wherein the cells have a mode value of not greater than 1.0 µm in their particle size distribution as measured by a laser particle size distribution analyzer, wherein the cells are obtainable by controlling pHs of media in a culturing step and processing steps of the lactic acid bacteria to a neutral range.
2. The cells as described above under 1, wherein the cells are obtainable by controlling the pHs of the media to 5 to 8.
3. The cells as described above under 1 or 2, wherein glucose is incorporated in the medium in the culture step of the lactic acid bacteria.
4. The cells as described above under 1, wherein the cells are obtainable by adding and dispersing a dispersant or excipient to a culture, which contains a medium with its pH controlled to a neutral range and cells, and then subjecting the resultant dispersion to freeze drying or spray drying.
5. The cells as described above under any one of 1 to 4, wherein the Lactobacillus lactic acid bacteria is Lactobacillus brevis.
6. The cells as described above under 5, wherein the Lactobacillus brevis is FERM BP-4693 strain.
7. A composition having immunopotentiating activity, including the cells as described above under any one of 1 to 6.
7. A food, drink, feed, cosmetic or drug including the cells as described above under to any one of 1 to 6, or the composition as described above under 7.
8. A process for producing the cells as described above under 1, wherein pHs of media in a culturing step and processing steps of the lactic acid bacteria are controlled to a neutral range.
9. The process as described above under 8, wherein the pHs of the media are controlled to 5 to 8.
10. The process as described above under 8, including adding and dispersing a dispersant or excipient to a culture, which contains a medium with its pH controlled to a neutral range and cells, and then subjecting the resultant dispersion to freeze drying or spray drying.

### ADVANTAGEOUS EFFECTS OF THE INVENTION

According to the present invention, even for usual lactic acid bacteria cells having a particle size greater than 1 µm, their particle size can be adjusted to 1.0 µm or smaller through its culture and processing at pHs in a neutral range.
The cells of the nano-sized lactic acid bacteria, which are obtainable as described above, are considered to be very useful cells, because they can enhance the IFN-α producing capacity from antigen presenting cells and can improve the immunopotentiating activity.

### BRIEF DESCRIPTION OF THE DRAWINGS

[FIG. 1] Diagrams illustrating correlations between the cell particle size of lactic acid bacteria and IL-12 and IFN-α producing capacities.
[FIG. 2] A diagram illustrating a correlation between IL-12 and IFN-α producing capacities as a result of stimulation by lactic acid bacteria.
[FIG. 3] A diagram comparing cell particle sizes.
[FIG. 4] Diagrams illustrating particle size distributions (A: frequency %, B: cumulative %) of a lactic acid bacteria powder.
[FIG. 5] A diagram illustrating cell particle size and IFN-α producing capacity of nano-sized lactic acid bacteria, "LABRE."

### BEST MODE FOR CARRYING OUT THE INVENTION

The present invention will hereinafter be described in further detail.
The cells of the nano-sized lactic acid bacteria according to the present invention have a mode value (particle size) of 1.0 µm or smaller in their particle size distribution.
In the present invention, the expression "a mode value in their particle size distribution" is a value serving as an index that indicates the size of the bacteria, and means a particle size at which the relative frequency becomes maximum in a particle distribution when the particle sizes of cells are measured.

Specific examples of lactic acid bacteria usable as sources for "the cells of the nano-sized lactic acid bacteria" in the present invention include Lactobacillus bacteria such as L. acidphilus, L. gasseri, L. mali, L. plantarum, L. buchneri, L. casei, L. johnsonii, L. gallinarum, L. amylovorus, L. brevis, L. rhamnosus, L. kefir, L. paracasei and L. crispatus.

The cells of the nano-sized lactic acid bacteria according to the present invention may be in the form of vial cells or dead cells. Vial cells may undergo a change in profile during delivery or display after their manufacture as a product, and therefore, dead cells which undergo no further change in profile are preferred.

It is known that, when the growth environment deteriorates during culture, lactic acid bacteria change in profile under its stress.
The present invention, therefore, controls the culture and processing conditions such that lactic acid bacteria is allowed to proliferate while maintaining it uniform in profile, and produces the nano-sized lactic acid bacteria having the above-mentioned mode value in its particle size distribution.
Described specifically, paying attention to the fact that lactic acid bacteria is charged positive (plus) at surfaces thereof as mentioned above, the membranes are stabilized by controlling the pHs in its culturing step and processing steps to a neutral range, thereby making it possible to avoid a double-cell form that fission cells remain fused together or re-adsorption of cells themselves.

As a method for "controlling the pHs to a neutral range," neutralization with an acid such as lactic acid or an alkali such as sodium hydroxide can be mentioned.
It is to be noted that the expression "controlling the pHs in its culturing step and processing steps to a neutral range" in the present invention means not only to control the pH of the culture step to the neutral range in advance but also to control the pHs to the neutral range in steps (processing steps) such as cell sterilization, washing and concentration after the completion of the culture.
The pHs in the culturing step and processing steps may be preferably from 5 to 8, more preferably from 5.5 to 7.5.

As an energy source in the nutrient composition of the medium, glucose is preferred for its highest energy availability. Its content may be controlled preferably to 5 to 10 wt% or so in the medium.
By employing as a culture endpoint the time point that glucose has been used up, it is possible to avoid a change in cell profile which would otherwise take place under a stress applied as a result of nutrient deprivation.

Further, "the cells of the nano-sized lactic acid bacteria" according to the present invention may preferably be in a form subjected to dispersion processing.
No particular limitation is imposed on the manner of the dispersion processing. For example, however, there can be mentioned a method that disperses the culture of the bacteria under wet conditions in a high-pressure homogenizer controlled at 150 kgf/cm² (1.5 MPa) or so.
In this case, it is preferred to add a known dispersant or excipient in the culture beforehand. By the dispersant or excipient, reaggregation of the cells can be efficiently prevented.
The amount of the dispersant or excipient to be added varies depending upon the properties of the cells, but may be preferably from 1 to 100-fold, more preferably from 2 to 20-fold in terms of weight relative to the cells.
Preferred dispersants or excipients can be trehalose, dextrin, skimmed milk, and the like.

When it is desired to eventually obtain as a powder "the cells of the nano-sized lactic acid bacteria" according to the present invention, it is preferred to conduct freeze drying or spray drying after applying treatment to the cells with a known dispersant, excipient or the like such that the cells do not undergo reaggregation. This treatment makes it possible to obtain a cell powder having excellent dispersibility in water.

The above-described cells of the nano-sized lactic acid bacteria according to the present invention have been reduced to a particle size on the order of nanometers (nm) not greater than 1.0 µm.
These cells remain to also have a cell particle size of 1.0 µm or smaller when prepared into a dry powder by the above-described method and re-suspended in a physiological digestive fluid. It is to be noted that the term "physiological digestive fluid" as used herein means an artificial gastric fluid or intestinal fluid prepared by a method known per se in the art.

The cells of the nano-sized lactic acid bacteria according to the present invention can be used as a product as they are. For improving their flavor and/or taste or forming them into a required shape or for a similar purpose, however, they are generally formulated into a final product by adding or mixing various ingredients and further mixing one or more flavors.
As the ingredients to be added or mixed, various carbohydrates, emulsifiers, sweeteners, sour seasonings, fruit extracts and the like can be mentioned.

More specifically, there can be mentioned sugars such as glucose, sucrose, fructose and honey; sugar alcohols such as sorbitol, xylitol, erythritol, lactitol and paratinit; and emulsifiers such as sucrose fatty acid esters, glycerin fatty acid esters and lecithin. Th1 inducers of excellent flavor and taste can also be obtained even when one or more of various vitamins such as vitamin A, vitamin Bs, vitamin C and vitamin E, herb extracts, cereal components, vegetable components, milk components and the like are mixed.

As flavors, there can be mentioned flavors such as yoghurt flavor, berry flavor, orange flavor, guince flavor, Japanese basil favor, citrus flavor, apple flavor, mint flavor, grape flavor, pear flavor, custard cream flavor, peach flavor, melon flavor, banana flavor, tropical fruit flavor, herb flavor, black tea flavor and coffee flavor. They can be used either singly or in combination. No particular limitation is imposed on the amount of each flavor to be added, but from the stand point of flavor, each flavor may be added preferably at 0.05 to 0.5 wt% or so, notably at 0.1 to 0.3 wt% or so.

The above-described cells of the nano-sized lactic acid bacteria according to the present invention can be formulated into any form such as a solid form or a liquid form. Described specifically, they can be formulated together with pharmaceutically-acceptable salts, excipients, preservatives, colors, corrigents and/or the like into products of various forms such as drinks, granules, tablets and capsules by methods known in the drug or food manufacturing field.

Further, the cells of the nano-sized lactic acid bacteria according to the present invention can also be used in health foods. The term "health foods" means foods intended for healthcare or health maintenance and promotion in a more positive sense than ordinary foods. Their forms may be liquid, semi-slid, or solid. Specific examples include confectioneries such as cookies, rice crackers, jellies, sweet bean jellies, yoghurt, and steamed or baked buns with bean-jam filling; cooling beverages; nutritional drinks; and soups.

Furthermore, the cells of the nano-sized lactic acid bacteria according to the present invention can also be used in skin external preparations in a variety of product forms such as lotions (cosmetic waters), cosmetic creams, emulsions, cosmetic waters, cosmetic packs, skin milks (emulsifiable concentrates), cosmetic gels, powders, lip creams, lipsticks, under makeup cosmetics, foundations, sun care cosmetics, bathwater additives, body shampoos, body rinses, soaps, cleansing foams, ointments, adhesive skin patches, jelly formulations, and aerosol formulations.

It is to be noted that to the cells of the nano-sized lactic acid bacteria according to the present invention, various ingredients and additives commonly employed in cosmetics, quasi drugs and drugs, such as those to be exemplified below, can also be suitably mixed as needed.
Specifically, it is possible to suitably mix humectants such as glycerin, petrolatum, urea, hyaluronic acid and heparin; ultraviolet absorbers or scatters such as PABA derivatives (paraaminobenzoic acid, "ESCALOL 507" (ISP Japan Ltd.), etc.), cinnamic acid derivatives (neoheparin, "PARSOL MCX" (DSM Nutrition Japan K.K.), "SUNGUARD B" (Shiseido Co., Ltd.), etc.), salicylic acid derivatives (such as octyl salicylate), benzophenone derivatives ("ASL-24," "ASL-24S" (Shonan Chemical Services, Inc.), etc.), dibenzoylmethane derivatives ("PARSOL A," "PARSOL DAM" (DSM Nutrition Japan K.K.), etc.), heteroring derivatives ("TINUVIN" series, etc.) and titanium oxide; metal scavengers such as disodium edentate, trisodium edentate, citric acid, sodium citrate, tartaric acid, sodium tartrate, lactic acid, malic acid, polysodium phosphate, sodium metaphosphate and gluconic acid; sebum inhibitors such as salicylic acid, sulfur, caffeine and tannin; antiseptic disinfectants such as benzalkonium chloride, benzethonium chloride and chlorohexidine gluconate; anti-inflammatories such as diphenhydramine chloride, tranexamic acid, guaiazulene, azulene, alantoin, Hinokitiol, glycyrrhizic acid and its salts, glycyrrhizic acid derivatives and glycylrrhetinic acid; vitamins such as vitamin A, vitamin Bs (B1, B2, B6, B12, B15), folic acid, nicotinic acids, pantothenic acids, biotin, vitamin C, vitamin Ds (D2, D3), vitamin E, ubiquinone and vitamin Ks (K1, K2, K3, K4): amino acids and derivatives thereof, such as aspartic acid, glutaminic acid, alanine, lysine, glycine, glutamine, serine, cysteine, cystin, tyrosine, proline, arginine and pyrrolidonecarboxylic acid; skin-lightening agents such as retinol, tocopherol acetate, magnesium ascorbate phosphate, ascorbic acid glucoside, arbutin, kojic acid, ellagic acid and placenta extract; antioxidants such as butylhydroxytoluene, butylhydroxyanisole and propyl gallate; astringent agents such as zinc chloride, zinc sulfate, zinc phenate, zinc oxide and aluminum potassium sulfate; sugars such as glucose, fructose, maltose, sucrose, trehalose, erythritol, mannitol, xylitol and lactitol; various plant extracts such as licorice, chamomile, horse chestnut, strawberry geranium, paeoniae radix, quince, scutellaria, phellodendron bark, coptis rhizome, Houttuyniae herba and ginkgo biloba; and in addition, oil ingredients, surfactants, thickeners, alcohols, powder ingredients, colors, and the like.

### EXAMPLES

The present invention will hereinafter be described more specifically based on Referential Examples and Examples. It should however be borne in mind that the present invention shall not be limited to the below-described Examples. It is to be noted that the particle size of each lactic acid bacteria was measured by a particle size distribution analyzer ("SALD-3100," Shimadzu Corporation). Further, IL-12 and IFN-α produced from macrophages as a result of stimulation by each lactic acid bacteria were assayed by a commercially-available ELISA kit.

### Referential Example 1

### [Preparation of dead cells]

As shown in Table 1, lactic acid bacteria which contribute to longevity were isolated from "Sugukizuke" in Kyoto, "Sunkizuke" in Nagano, Mariami and Matsoni-like in Republic of Georgia, fermented mare's milk in Mongolia, and various fermented dairy products, and were cultured in an MRS medium at 36.5°C for 48 hours without controlling the pH during the culture. After completion of the culture, each culture was heated at 80°C for 10 minutes to subject it to sterilization treatment. The cells were washed with PBS and were formulated to give a cell concentration of 10 mg/mL.
It is to be noted that the abbreviation "LBR" in the table indicates the Lactobacillus brevis strain, FERM BP-4693.

### [Correlations between cell particle size and IL-12 and IFN-α producing capacities]

Correlations between the cell particle size of lactic acid bacteria and L-12 and IFN-α producing capacities are illustrated in FIG. 1A and FIG. 1B, respectively.
It has been ascertained that the producing capacity is significantly enhanced for both of the cytokines L-12 and IFN-α when the size (particle size) of lactic acid bacteria becomes smaller to 1 µm or so (see Table 1 and FIGS. 1A and 1B).
As illustrated in FIG. 2, however, some strains have been found to show a negative correlation between IL-12 producing capacity and IFN-α producing capacity. For example, there have been recognized such characteristic features that strains such as EF, KH1 and KH3 are high in IL-12 producing capacity but low in IFN-α producing capacity, strains such as LL12 and ML4 are conversely low in IL-12 producing capacity but high in IFN-α producing capacity, and on the other hand, SNK is relatively high in both of IL-12 producing capacity and IFN-α producing capacity.

**Table 1**

| Sources, particle sizes and cytokine producing ability of lactic acid bacteria | | | | | |
|---|---|---|---|---|---|
| Strain name | | Source | Particle size (µm) | IL-12 (pg/ml.) | IFN-α (pg/mL) |
| (Lactic acid bacteria: 10 µg/mL) | | | | | |
| | KH13 | Fruit | 1.15 | 6266 | 15.8 |
| | LL12 | Fermented mare's milk (Mongolia) | 1.22 | 1350 | 41.6 |
| | EF | Human intestinal tract | 1.22 | 6266 | 16.0 |
| | SNK | Sunkizuke (Nagano) | 1.51 | 3468 | 28.8 |
| | KH1 | Fruit | 1.51 | 5294 | 10.4 |
| | DL1 | Mariami (Republic of Georgia) | 1.69 | 2223 | 20.8 |
| | CF4 | Yoghurt (Caspian Sea) | 1.69 | 2741 | 17.3 |
| | ML4 | Matsoni-like (Republic of Georgia) | 1.89 | 757.3 | 29.3 |
| | ST100 | Dairy product | 2.11 | 3735 | 14.0 |
| | LC12 | Dairy product | 2.35 | 3863 | 14.8 |
| | LGG | Dairy product | 2.93 | 2323 | 12.7 |
| | DL2 | Mariami (Republic of Georgia) | 4.55 | 2185 | 14.8 |
| | LBR | Sugukizuke (Kyoto) | 8.80 | 1583 | 16.8 |

| (Positive controls) | | | | | |
|---|---|---|---|---|---|
| | OK-432 (5 µg/mL) | | | 4137 | 3.4 |
| | H1N1 influenza (70 µg/mL) | | | | 22.6 |

### Comparative Example 1

### [Preparation of nano-sized lactic acid bacteria EF]

A Lactic acid bacteria strain, Enterococcus faecalis EF, was cultured at 36.5°C in a known nutrient medium with 5 wt% of glucose added therein while controlling the pH at 6.5 with a 20 wt% aqueous solution of sodium hydroxide during the culture. The time point at which the glucose had been used up was employed as a culture endpoint (culturing step).
After completion of the culturing, the culture was heated at 80°C for 10 minutes to subject it to sterilization treatment. The cells were then washed with PBS and were formulated to give a cell concentration of 10 mg/mL (processing steps). During the processing steps, the pH was maintained at 6.5.

### [Comparison of particle sizes]

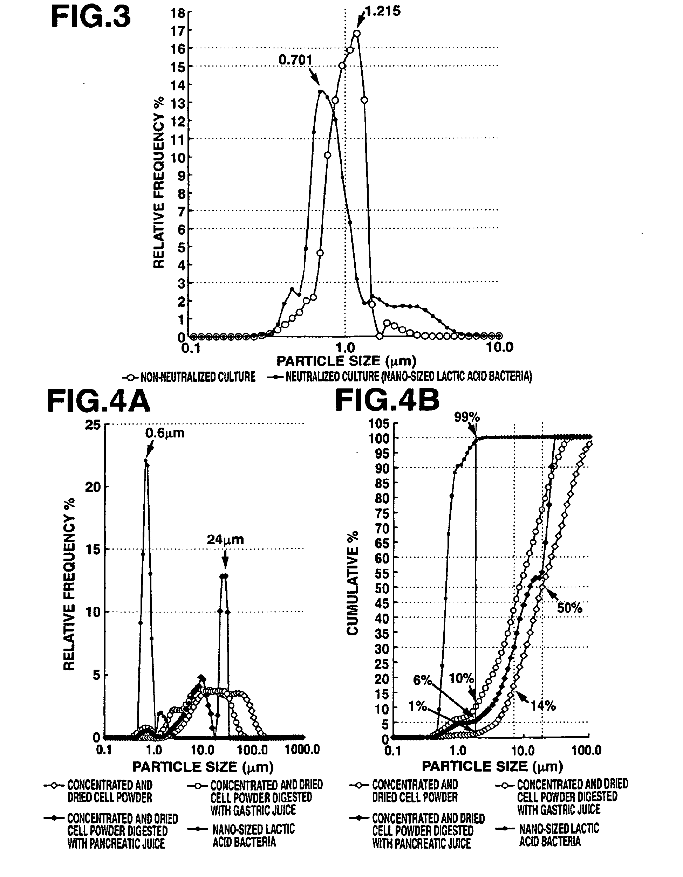

The cell particle sizes of lactic acid bacteria EF prepared in Referential Example 1 and Example 1 were measured. The results are illustrated in FIG. 3.
As illustrated in FIG. 3, it is appreciated that, in contrast to the cell particle size of 1.215, i.e., greater than 1 µm by the non-neutralized culture, the cell particle size by the neutralized culture was 0.701, i.e., smaller than 1.0 µm

### Referential Example 2

### [Preparation of dry powder of dead cells]

Following the procedure of Example 1, the Lactic acid bacteria strain, Enterococcus faecalis EF, was cultured. The size of the lactic acid bacteria itself was 0.6 µm (FIG. 4A), and the cumulative distribution of particles of 2 µm and smaller which can selectively induce cytokines was 99% (FIG. 4B). From the culture, cells were concentrated, and without addition of any excipient, the concentrate was spray-dried into a powder.

### [Comparison of particle sizes]

The powder prepared as described above was dispersed again in water. As a result, a particle size distribution which was gentle up to a particle size of 100 µm was shown as illustrated in FIG. 4A. As illustrated in the cumulative distribution in FIG. 4B, the particle size distribution was broken down into about 50% of particles of up to 20 µm that pass through Peyer's patches, about 14% of particles of 3 to 7 µm that induce Th2 cells, and as little as 1% of particles of 2 µm and smaller that induce Th1 cells. This indicates the admixture of particles that induce both of a Th1 response and a Th2 response, and therefore, is by no means considered to be a physiologically-preferable situation.

Assuming in vivo digestive activities, the powder was also treated with an artificial gastric fluid and intestinal fluid prepared in a manner known per se in the art. Even in those cases, the cumulative distribution of particles of 2 µm and smaller that induce Th1 cells increased only to 10% at the greatest (FIG. 4).
Such a cumulative particle size distribution is apprehensive of yielding a result that the valuable function inherent to lactic acid bacteria may not be fully exhibited (as little as 10% or so at the greatest).

### Example I

### [Preparation of nano-sized lactic acid bacteria, Lactobacillus brevis]

A Lactic acid bacteria strain, Lactobacillus brevis BP-4693, was cultured at 36.5°C in a known nutrient medium with 5 wt% of glucose added therein while controlling the pH at 6.5 with a 20 wt% aqueous solution of sodium hydroxide during the culture. The time point at which the consumption of glucose had been completed was employed as a culture endpoint (culturing step).
After completion of the culturing, the culture was heated at 80°C for 10 minutes to subject it to sterilization treatment. The cells were then washed with PBS. Dextrin was added as an excipient in an amount four times as much as the cells in terms of weight. After the mixture was dispersed in a mixer, the dispersion was freeze-dried to prepare a sample. The sample was suspended again in PBS to give a cell concentration of 10 mg/ml (processing steps). During the processing steps, the pH was maintained at 6.5.

### [Comparison of cell particle sizes and IFN-α producing capacities]

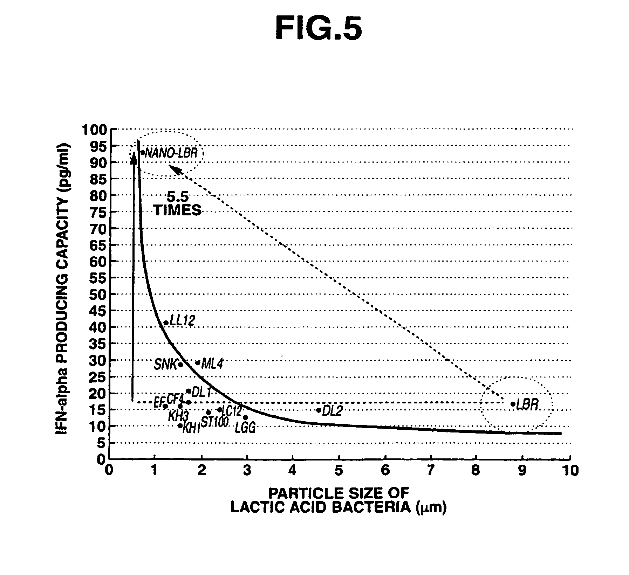

The cell particle sizes and IFN-α producing capacities of Lactobacillus brevis BP-4693 prepared in Referential Example 1 and Example 1 were measured. The results are illustrated in FIG. 5.
As illustrated in FIG. 5, it is appreciated that in the case of the non-neutralized culture (indicated as "LBR"), the cell particle size and IFN-α producing capacity were 8.8 µm and 16.8 pg/ml, respectively, and in the case of the neutralized culture (indicated as "NANO-LBR"), on the other hand, the cell particle size was reduced to 0.7 µm, i.e., smaller than 1.0 µm and the IFN-α producing capacity was 92.9 pg/ml, namely, was enhanced to 5.5 times compared with that in the case of the non-neutralized culture.

From the above results, it has been confirmed that the particle size of cells can be reduced to 1.0 µm or smaller by controlling the pH to a neutral range in culture and processing steps, a cell powder having excellent dispersibility can be obtained by adding an excipient to the cells in an amount approximately 4 times as much as the cells in terms of weight, subjecting the mixture to dispersing processing and then freeze-drying the dispersion, and with the powder, interferon-α can be efficiently produced from macrophages.

## Claims

1. Cells of nano-sized lactic acid bacteria belonging to Lactobacillus having a mode value of not greater than 1.0 µm in their particle size distribution as measured by a laser particle size distribution analzer, wherein the cells are obtainable by controlling pHs of media in a culturing step and processing steps of the lactic acid bacteria to a neutral range.

2. The cells according to claim 1, wherein the cells are obtainable by controlling the pHs of the media to 5 to 8.

3. The cells according to claim 1 or 2, wherein glucose is incorporated in the medium in the culture step of the lactic acid bacteria.

4. The cells according to claim 1, wherein the cells are produced by a process comprising a culturing step and processing steps, said processing steps comprising
adding a dispersant or excipient to a culture containing a medium with its pH controlled to a neutral range and cells,
dispersing the cells, and then
subjecting the resultant dispersion to freeze drying or spray drying.

5. The cells according to any one of the preceding claims, wherein the Lactobacillus lactic acid bacteria is selected from Lactobacillus casei, Lactobacillus brevis, and Lactobacillus kefir.

6. The cells according to claim5, wherein the Lactobacillus lactic acid bacteria is Lactobacillus brevis.

7. The cells according to claim 6, wherein the Lactobacillus brevis is FERM BP-4693 strain.

8. The cells according to claim 1, wherein the cells remain to have a particle size of 1.0 µm or smaller when the cells are re-suspended in a physiological digestive fluid.

9. A composition having immunopotentiating activity, comprising the cells according to any one of claims 1 to 8.

10. A food, drink, feed, cosmetic or drug comprising the cells according to any one of claims 1 to 8 or the composition according to claim 9.

11. A process for producing cells of nano-sized lactic acid bacteria belonging to Lactobacillus, wherein pHs of media in a culturing step and processing steps of the lactic acid bacteria are controlled to a neutral rangethereby obtaining cells of the bacteria having a mode value of not greater than 1.0 µm in their particle size distribution.

12. The process according to claim 11, wherein the pHs of the media are controlled to 5 to 8.

13. The process according to claim 11or claim 12, wherein glucose is incorporated in the medium in the culture step of the lactic acid bacteria.

14. The process according to claim 11, wherein the processing steps comprise adding a dispersant or excipient to a culture containing a medium with its pH controlled to a neutral range and cells, dispersing the cells, and then subjecting the resultant dispersion to freeze drying or spray drying.

15. The process according to any one of claims 11 to 14, wherein the lactic acid bacteria is Lactobacillus brevis.

16. The process according to claim 15, wherein the Lactobacillus brevis is FERM BP-4693 strain.

17. The process according to any one of claims 11 to 16, wherein the cells remain to have a cell particle size of 1.0 µm or smaller when the cells are re-suspended in a physiological digestive fluid.

18. The process according to any one of claims 11 to 17, wherein the processing steps comprise a washing step and a concentration step after completion of a culture.

19. The process according to any one of claims 11 to 18, wherein the processing steps comprise a sterilisation step.

## Patentansprüche

1. Zellen von Milchsäurebakterien in Nano-Größe der Gattung Lactobacillus mit einem Wert von nicht mehr als 1,0µm an Teilchengrößenverteilung bei Messung durch einen Laser-Teilchengrößenverteilungsanalysator, worin die Zellen durch Steuern des pH von Medien in einem Kultivierungsschritt und Verarbeitungsschritten der Milchsäurebakterien zu einem neutralen Bereich erhaltbar sind.

2. Zellen nach Anspruch 1, worin die Zellen durch Steuern des pH der Medien auf 5 bis 8 erhaltbar sind.

3. Zellen nach Anspruch 1 oder 2, worin beim Verarbeitungsschritt der Milchsäurebakterien Glukose in das Medium inkorporiert wird.

4. Zellen nach Anspruch 1, worin die Zellen durch ein Verfahren erzeugt werden, das einen Kultivierungsschritt und Verarbeitungsschritte umfasst, wobei die Verarbeitungsschritte Folgendes umfassen:
Zugabe eines Dispergators oder Exzipienten zu einer Kultur, die ein Medium, dessen pH auf einen neutralen Bereich gesteuert ist, und Zellen enthält,
Dispersion der Zellen und anschließend
Behandeln der sich ergebenden Dispersion durch Gefriertrocknung oder Sprühtrocknung.

5. Zellen nach einem der vorangegangenen Ansprüche, worin die Lactobacillus-Milchsäurebakterien aus der Gruppe von Lactobacillus casei, Lactobacillus brevis und Lactobacillus kefir ausgewählt sind.

6. Zellen nach Anspruch 5, worin die Lactobacillus-Milchsäurebakterien Lactobacillus brevis sind.

7. Zellen nach Anspruch 6, worin der Lactobacillus brevis vom FERM BP-4693-Stamm ist.

8. Zellen nach Anspruch 1, worin die Zellen eine Teilchengröße von 1,0µm oder kleiner beibehalten, wenn die Zellen in einem physiologischen Verdauungsfluid resuspendiert werden.

9. Zusammensetzung, die immunopotenzierende Aktivität aufweist, wobei die Zusammensetzung die Zellen nach einem der Ansprüche 1 bis 8 umfasst.

10. Nahrungsmittel, Getränk, Kosmetikum oder Pharmakon, das die Zellen nach einem der Ansprüche 1 bis 8 oder die Zusammensetzung nach Anspruch 9 erfasst.

11. Verfahren zum Erzeugen von Zellen von Milchsäurebakterien in Nano-Größe der Gattung Lactobacillus, worin der pH von Medien in einem Kultivierungsschritt und Verarbeitungsschritten der Milchsäurebakterien auf einen neutralen Bereich gesteuert wird, wodurch Zellen der Bakterien erhalten werden, die einen Wert von nicht mehr als 1,0µm an Teilchengrößenverteilung aufweisen.

12. Verfahren nach Anspruch 11, worin der pH der Medien auf 5 bis 8 gesteuert wird.

13. Verfahren nach Anspruch 11 oder 12, worin beim Kultivierungsschritt der Milchsäurebakterien Glukose in das Medium inkorporiert wird.

14. Verfahren nach Anspruch 11, worin die Verarbeitungsschritte die Zugabe eines Dispergators oder Exzipienten zu einer Kultur umfassen, die ein Medium, dessen pH auf einen neutralen Bereich gesteuert wird, und Zellen umfasst, wobei die Zellen dispergiert werden und die sich ergebende Dispersion einer Gefriertrocknung oder Sprühtrocknung unterzogen wird.

15. Verfahren nach einem der Ansprüche 11 bis 14, worin die Milchsäurebakterien Lactobacillus brevis sind.

16. Verfahren nach Anspruch 15, worin der Lactobacillus brevis vom FERM BP-4693-Stamm ist.

17. Verfahren nach einem der Ansprüche 11 bis 16, worin die Zellen eine Teilchengröße von 1,0µm oder kleiner beibehalten, wenn die Zellen in einem physiologischen Verdauungsfluid resuspendiert werden.

18. Verfahren nach einem der Ansprüche 11 bis 17, worin die Verarbeitungsschritte nach Fertigstellung einer Kultur einen Waschschritt und einen Konzentrierungsschritt umfassen.

19. Verfahren nach einem der Ansprüche 11 bis 18, worin die Verarbeitungsschritte einen Sterilisierungsschritt umfassen.

## Revendications

1. Cellules de bactéries d'acide lactique à l'échelle nano appartenant à Lactobacillus et ayant une valeur modale n'excédant pas 1,0 µm en ce qui concerne leur distribution de taille de particules telle que mesurée par un analyseur laser de distribution de taille de particules, où les cellules peuvent être obtenues en contrôlant les pH de milieux au cours d'une étape de culture et d'étapes de traitement des bactéries d'acide lactique dans une plage neutre.

2. Cellules selon la revendication 1, où les cellules peuvent être obtenues en contrôlant les pH des milieux entre 5 et 8.

3. Cellules selon la revendication 1 ou 2, où du glucose est incorporé dans le milieu au cours de l'étape de culture des bactéries d'acide lactique.

4. Cellules selon la revendication 1, où les cellules sont produites par un procédé qui comprend une étape de culture et des étapes de traitement, lesdites étapes de traitement consistant à
ajouter un agent de dispersion ou un excipient à une culture contenant un milieu dont le pH est contrôlé dans une plage neutre et des cellules,
disperser les cellules, puis
soumettre la dispersion résultante à une lyophilisation ou à un séchage par pulvérisation.

5. Cellules selon l'une quelconque des revendications précédentes, où les bactéries d'acide lactique Lactobacillus sont sélectionnées parmi Lactobacillus casei, Lactobacillus brevis, et Lactobacillus kefir.

6. Cellules selon la revendication 5, où les bactéries d'acide lactique Lactobacillus sont Lactobacillus brevis.

7. Cellules selon la revendication 6, où Lactobacillus brevis est la souche FERM BP-4693.

8. Cellules selon la revendication 1, où les cellules doivent conserver une taille de particules de 1,0 µm ou moins lorsque les cellules sont remises en suspension dans un liquide digestif physiologique.

9. Composition ayant une activité d'immuno-potentialisation, comprenant les cellules selon l'une quelconque des revendications 1 à 8.

10. Aliment, boisson, nourriture, produit cosmétique ou médicament comprenant les cellules selon l'une quelconque des revendications 1 à 8 ou la composition selon la revendication 9.

11. Procédé pour produire des cellules de bactéries d'acide lactique à l'échelle nano appartenant à Lactobacillus, où les pH de milieux au cours d'une étape de culture et d'étapes de traitement des bactéries d'acide lactique sont contrôlés dans une plage neutre, ce qui permet ainsi d'obtenir des cellules des bactéries ayant une valeur modale n'excédant pas 1,0 µm en ce qui concerne leur distribution de taille de particules.

12. Procédé selon la revendication 11, dans lequel les pH des milieux sont contrôlés entre 5 et 8.

13. Procédé selon la revendication 11 ou la revendication 12, dans lequel du glucose est incorporé dans le milieu au cours de l'étape de culture des bactéries d'acide lactique.

14. Procédé selon la revendication 11, dans lequel les étapes de traitement consistent à ajouter un agent de dispersion ou un excipient à une culture contenant un milieu dont le pH est contrôlé dans une plage neutre et des cellules, à disperser les cellules, puis à soumettre la dispersion résultante à une lyophilisation ou à un séchage par pulvérisation.

15. Procédé selon l'une quelconque des revendications 11 à 14, dans lequel les bactéries d'acide lactique sont Lactobacillus brevis.

16. Procédé selon la revendication 15, dans lequel Lactobacillus brevis est la souche FERM BP-4693.

17. Procédé selon l'une quelconque des revendications 11 à 16, dans lequel les cellules doivent conserver une taille de particules cellulaires de 1,0 µm ou moins lorsque les cellules sont remises en suspension dans un liquide digestif physiologique.

18. Procédé selon l'une quelconque des revendications 11 à 17, dans lequel les étapes de traitement comprennent une étape de lavage et une étape de concentration après la fin d'une culture.

19. Procédé selon l'une quelconque des revendications 11 à 18, dans lequel les étapes de traitement comprennent une étape de stérilisation.
